# EUROPEAN PATENT APPLICATION

(11) **EP 1 038 533 A1**
(43) Date of publication of application: **27.09.2000**
(21) Application number: 98932529.5
(22) Date of filing: 15.07.1998
(51) Int. Cl.: A61K 45/00, A61K 31/16

(54) **MEDICINAL COMPOSITIONS FOR TREATING IMMUNODEFICIENCY DISEASES**

(30) Priority: 18.07.1997 JP 20837997
(71) Applicant: Nippon Kayaku Kabushiki Kaisha, Tokyo 102-8172 (JP)
(72) Inventor: YOSHIDA, Masao, Tokyo 204-0021 (JP); NEMOTO, Kyuichi, Tochigi 329-2163 (JP); DUREN, Erol, Besiktas, 80680 Istanbul (TR)
(74) Representative: Gille Hrabal Struck Neidlein Prop Roos
(86) International application number: JP9803175
(87) International publication number: WO9903504

(57) **Abstract**

Spergualin-related compounds such as 15-deoxyspergualin and pharmacologically acceptable salts thereof possess an activity of suppressing the function of monocyte or macrophage are useful as drugs for the treatment of immunodeficiency accompanied by a reduced CD4⁺ T cell level as observed in HIV infection.

## Description

### TECHNICAL FIELD

The present invention relates to a pharmaceutical composition for the treatment of immunodeficiency diseases such as acquired immunodeficiency syndrome (AIDS) caused by HIV (human immunodeficiency virus) infections, especially immunodeficiency diseases with reduction in a CD4⁺ T cell level.

### BACKGROUND ART

AIDS is an immunodeficiency disease developed by HIV infections. At the outbreak of AIDS, a marked reduction of the CD4⁺ T cell level is observed (Annual Review, MEN-EKI (immunity), 1996, page 211). Currently, anti-HIV agents such as AZT (Azidothymidine) are employed for the treatment of AIDS.

Immunosuppressive agents are generally available for the prevention of rejection triggered by organ or tissue transplantation or for the prevention of an excessive immune response observed in autoimmune diseases, etc. These immunosuppressive agents are considered to worsen immunodeficiency so that basically they are not applied to immunodeficiency diseases.

It is known that one of spergualin derivatives, 15-deoxyspergualin, isolated from its producing bacteria belonging to the genus Bacillus, exhibits anti-tumor and immunosuppressive activities (Japanese Patent KOKAI Nos. 58-62152 and 61-129119).

In the treatment with anti-AIDS agents presently available, the therapeutic effects are becoming noticeable by combined therapy; on the other hand, these anti-AIDS agents are disadvantages in that side effects are rather serious and their therapeutic effects are not fully satisfactory. It is thus earnestly desired to develop a novel and highly safe anti-AIDS agent.

### DISCLOSURE OF INVENTION

The present inventors have made extensive studies to develop a new type of anti-AIDS agent. As a result, it has been discovered that by administering 15-deoxyspergualin, which possesses an activity of suppressing the function of monocyte or macrophage, to HIV-infected patients with a markedly reduced CD4⁺/CD8⁺ level and complaining of fatigue or malaise, the conditions are improved, or example, the CD4⁺/CD8⁺ level can be strikingly improved, patients are recovered from fatigue or malaise, etc. The present invention has thus been accomplished. Since it is considered that reduction in the level of CD4⁺ T cells in HIV-infected patients as well as increase in the amount of HIV would be caused by the action of monocyte or macrophage of HIV-infected patients, it is highly likely that by suppressing the function of monocyte or macrophage, the reduction of CD4⁺ T cells is prevented and thus CD4⁺ T cells increase, resulting in a markedly improved CD4⁺/CD8⁺ level. Further, the reduction in the amount of HIV would be also resulted from the suppression on the function of monocyte or macrophage which plays an important role in HIV production.

Therefore, these results indicate that immunosuppressive compounds for preventing the function of monocyte or macrophage and pharmacologically acceptable salts thereof are effective for the treatment of immunodeficiency diseases with reduction in a CD4⁺ T cell level.

Therefore, an object of the present invention is to provide a pharmaceutical composition for the treatment of immunodeficiency diseases accompanied by a reduced CD4⁺ T cell level, comprising as an effective ingredient an immunosuppressive compound having the action of suppressing the function of monocyte or macrophage or a pharmacologically acceptable salt thereof.

In a preferred aspect of the aforesaid composition, an immunodeficiency disease accompanied by a reduced CD4⁺ T cell level is caused by human immunodeficiency virus.

In another preferred aspect of the composition, the immunosuppressive compound is spergualin or a spergualin-related compound having an immunosuppressive activity.

In a further preferred aspect of the composition, the spergualin-related compound immunosuppressive compound is 15-deoxyspergualin.

Another object of the present invention is to provide a pharmaceutical composition for the treatment of HIV infections or for the prevention of developing HIV infections, comprising as an effective ingredient 15-deoxyspergualin or a pharmacologically acceptable salt thereof.

A further object of the present invention is to provide use of an immunosuppressive compound having the activity of suppressing the function of monocyte or macrophage for preparing a pharmaceutical composition for the treatment of immunodeficiency disease accompanied by a reduced CD4⁺ T cell level, comprising as an effective ingredient said immunosuppressive compound or a pharmacologically acceptable salt thereof.

A still further object of the present invention is to provide use of a pharmaceutical composition for the treatment of HIV infections or for the prevention of developing HIV infections, comprising as an effective ingredient 15-deoxyspergualin or a pharmacologically acceptable salt thereof.

A still further object of the present invention is to provide a method for treating an immunodeficiency disease accompanied by a reduced CD4⁺ T cell level, which comprises administering to the patient an effective dose of an immunosuppressive compound having the activity of suppressing the function of monocyte or macrophage or a pharmacologically acceptable salt thereof.

A still further object of the present invention is to provide a method for treating HIV infections or preventing the development of HIV infections, which comprises administering to the patient an effective dose of 15-deoxyspergualin or a pharmacologically acceptable salt thereof.

### BEST MODE FOR CARRYING OUT THE INVENTION

Representative examples of the immunosuppressive compound of the present invention for suppressing the function of monocyte or macrophage include the following compounds.

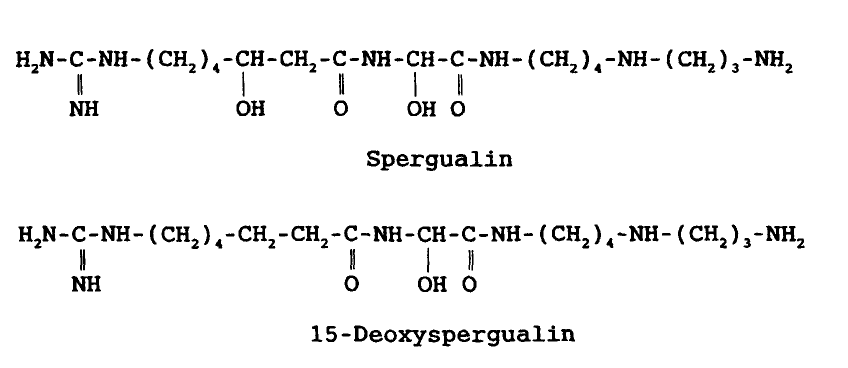

Both spergualin and 15-deoxyspergualin are compounds isolated from the producing bacteria belonging to the genus Bacillus. These compounds are known to exhibit anti-tumor and immunosuppressive activities (Japanese Patent KOKAI Nos. 57-48957, 58-62152 and 61-129119).

In addition to spergualin and 15-deoxyspergualin, other spergualin-related compounds disclosed in, e.g., Japanese Patent KOKAI Nos. 59-42356. 60-185758 and 62-48660 can also be used as the immunosuppressive compound which is the effective ingredient for the composition of the present invention.

More specifically, the spergualin-related compounds disclosed in Japanese Patent KOKAI No. 59-42356 are represented by general formula (I): 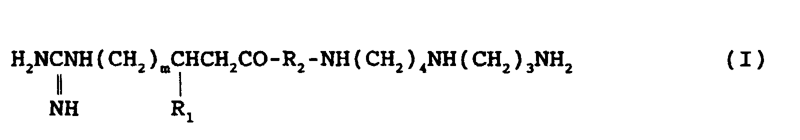 wherein R₁ represents a hydrogen atom, hydroxy or an aliphatic acyloxy group having 1 to 10 carbon atoms; R₂ represents a residue obtained by removing hydroxy group from carboxyl group and a hydrogen atom from the α- or ω-amino group of an α- or ω-amino acid and R₂ is combined with the carbonyl or amino adjacent thereto to form an acid amide bond; and m represents an integer of 4 to 6; and salts thereof.

The spergualin-related compounds of general formula (I) which can be used as the active component of the present invention are described below in more detail. In general formula (I) above, R₁ represents a hydrogen atom, hydroxy or an aliphatic acyloxy group having 1 to 10 carbon atoms; representative examples of the aliphatic acyloxy group are formyloxy, acetoxy, propionyloxy, butanoyloxy, pentanoyloxy, hexanoyloxy, heptanoyloxy, octanoyloxy, nonanoyloxy or decanoyloxy, preferably a lower alkylacyloxy group having 1 to 4 carbon atoms.

In general formula (I) above, R₂ represents a residue obtained by removing hydroxy group from carboxyl group and a hydrogen atom from α- or ω-amino group of an α- or ω-amino acid (hereinafter merely referred to as an amino acid residue). Any amino acid residue can be used without any particular limitation so long as the residue is derived from known α- or ω-amino acid.

Where the amino acid residue contains an optically active carbon(s), the residue can be any of L-, D- and DL-forms.

Examples of the amino acid residue include groups represented by formula: 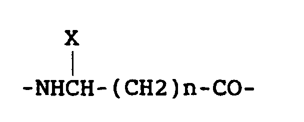 wherein X represents a hydrogen atom or a straight or branched alkyl group having 1 to 6 carbon atoms (wherein the alkyl moiety may be substituted with hydroxyl, a lower alkoxy group, carboxyl, a lower alkyloxycarbonyl group, amino, guanidino, phenyl, a hydroxyl-substituted phenyl group, imidazole, indole, mercapto or a lower alkylmercapto group, etc.; and n represents 0 or an integer of 1 to 5); or groups represented by formula: 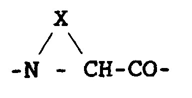 wherein X represents an alkylene group having 3 carbon atoms which may be substituted with hydroxyl. Examples of specific amino acids corresponding to the groups described above are given below: an α-amino acid such as glycine, alanine, α-aminobutyric acid, proline, valine, norvaline, isoleucine, alloisoleucine, leucine, norleucine, serine, homoserine, threonine, allothreonine, O-methylserine, O-ethylserine, O-methylhomoserine, O-ethylhomoserine, O-methylthreonine, O-ethylthreonine, O-methylallothreonine, O-ethylallothreonine, ornithine, lysine, aspartic acid, glutamic acid, asparagine, glutamine, arginine, phenylalanine, tyrosine, histidine, tryptophane, cysteine, homocysteine, S-methylcysteine, S-ethylcysteine, methionine, ethionine, etc.; and other amino acids such as β-alanine, γ-aminobutyric acid, δ-aminovaleric acid, ε-aminocaproic acid, etc.

In the compounds shown by general formula (I), m is an integer of 4 to 6; where the 15-position is substituted, its steric configuration may be any one of S-, R- and RS-configurations.

Among these compounds, preferred are those wherein m is 4 or 6 and the amino acid residue is a residue of serine, β-alanine, γ-aminobutanoic acid, arginine or phenylalanine.

Examples of the compounds Y17 disclosed in Japanese Patent KOKAI No. 60-185758 supra include the spergualin-related compound containing phenylene and represented by general formula (II): 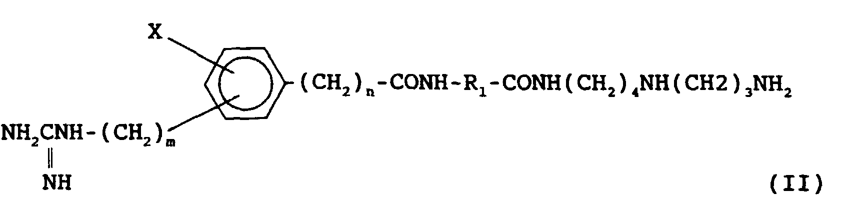 wherein R₁ represents a lower alkylene group which may be substituted with hydroxymethyl; X represents a hydrogen atom or a halogen atom; and m and n is 0 or an integer of 1 to 5; or salts thereof.

In the spergualin-related compounds of general formula (II), R₁ represents a lower alkylene group which may be substituted with a hydroxymethyl group, such as methylene, ethylene, propylene, or hydroxymethylmethylene, 1-hydroxymethylethylene, 2-hydroxymethylethylene, 1,2-di(hydroxymethyl)ethylene, 1-hydroxymethylpropylene, 2-hydroxymethylpropylene, 3-hydroxymethylpropylene, 1,2-di(hydroxymethyl)propylene, 1,3-di(hydroxymethyl)propylene, 2,3-di(hydroxymethyl)propylene, 1,2,3-tri(hydroxymethyl)propylene, etc.; X represents a hydrogen atom or a halogen atom such as chlorine, bromine, fluorine or iodine; and m and n represents 0 or an integer of 1 to 5, wherein the positional relationship between these methylene groups is not particularly limited and may be any of orth-, meta- and para-positions.

Further examples of the spergualin-related compounds include those disclosed in Japanese Patent KOKAI No. 62-48660 and represented by general formula (III): 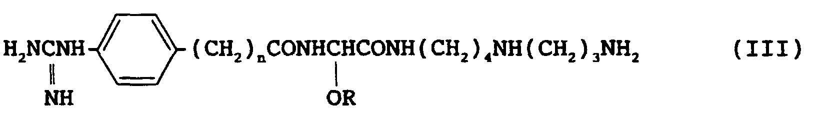 wherein R represents a hydrogen atom or a lower alkyl group and n represents an integer of 3 to 5.

In general formula (III), specific examples of the lower alkyl group shown by R are a lower alkyl group having 1 to 6 carbon atoms such as methyl, ethyl, propyl, butyl, pentyl, hexyl, etc.

Still further examples of the spergualin-related compounds include compounds obtained by substituting the terminal amino group of spergualin further with an amino acid residue, an acyl group, etc., such as compounds described in Japanese Patent KOKAI No. 1-90164. More specifically, those spergualin-related compounds are represented by general formula (IV): 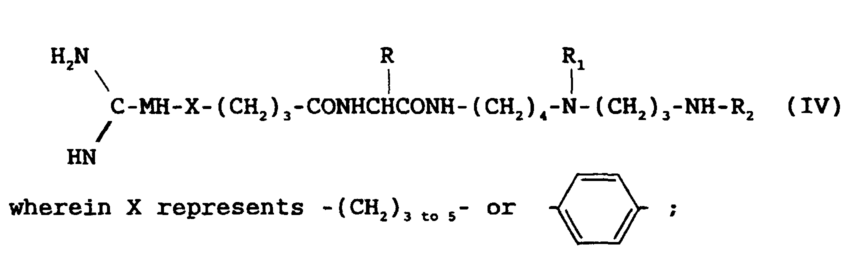 R represents -H or -CH₂-OH; R₁ and R₂ represent a residue obtained by removing hydroxy from the carboxyl group of an amino acid or a peptide.

In general formula (IV), specific examples of R₁ and R₂ are residues obtained by removing hydroxy from the carboxyl group of amino acids or peptides. Examples of the amino acids and peptides are given below; the steric configuration of amino acid residues shows L- or DL-form, except for glycine, β-alanine and γ-aminobutyric acid.

### (1) Amino acids

alanine, arginine, ornithine, aspartic acid, asparagine, cysteine, cystine, glutamic acid, glutamine, pyroglutamic acid, glycine, histidine, lysine, proline, hydroxyproline, isoleucine, leucine, methionine, phenylalanine, phenyl-substituted phenylalanine, serine, threonine, tryptophane, homoserine, tyrosine, valine, phenylglycine, p-hydroxyphenylglycine, 4-hydroxymethyl-3-hydroxyphenylglycine, β-alanine, γ-aminobutyric acid, 3-amino-2-hydroxy-4-phenylbutyric acid, etc.

### (2) Peptides

Preferred are di- or tripeptides obtained by condensing 2 or 3 of single or different amino acids described in (1) above. Examples of di- or tripeptides are alanylalanine, leucylleucine, valylvaline, phenylalanylphenylalanine, tyrosyltyrosine, phenylglycylphenylglycine, glycylglycine, isoleucylisoleucine, leucylphenylalanine, phenylalanylleucine, leucylphenylglycine, phenylglycylleucine, glycylglycylglycine, phenylglycylphenylglycylphenylglycine, phenylalanylphenylalanylphenylalanine, leucylleucylleucine, etc.

More preferred examples of the amino acid or peptide are phenylglycine, phenylalanine, leucine, aspartic acid, tryptophane, alanine and peptides formed by condensation of 2 or 3 of these amino acids.

Further examples of the spergualin-related compounds include 15-deoxyspergualin analogues wherein the backbone of 15-deoxyspergualin is partly modified, e.g., compounds described in Japanese Patent KOKAI Nos. 6-293728, 9-77733 and 8-41007 and Japanese Patent PCT KOKAI No. 9-511765. More specifically, the spergualin-related compounds are those described in Japanese Patent KOKAI No. 6-293728 and shown by general formula (V): 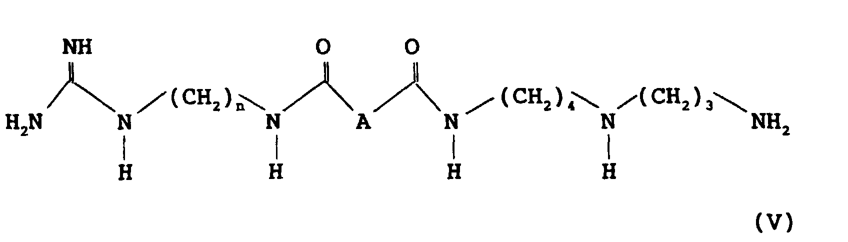 wherein n is 6 or 8; A represents a single bond, CH₂, CH(OH), CHF, CH(OCH₃), CH₂NH or CH₂O group; or salts thereof.

Furthermore, the spergualin-related compounds are those described in Japanese Patent KOKAI No. 9-77733 and shown by general formula (VI): 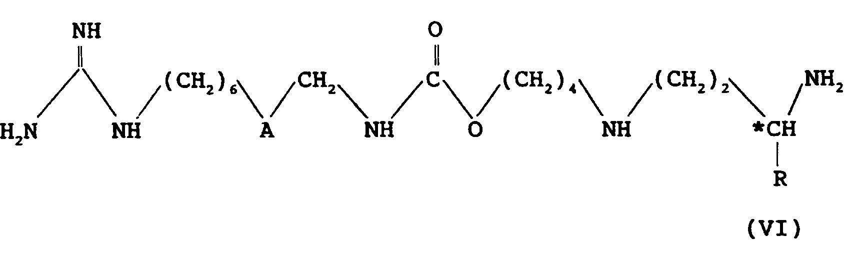 wherein A represents -CO-NH- or -NH-CO- group; R represents a hydrogen atom or CH₃; and *C represents an asymmetric carbon of (R,S) or (R) configuration when R is not a hydrogen atom; or salts thereof.

Furthermore, the spergualin-related compounds are those described in Japanese Patent KOKAI No. 8-41007 and shown by general formula (VII): 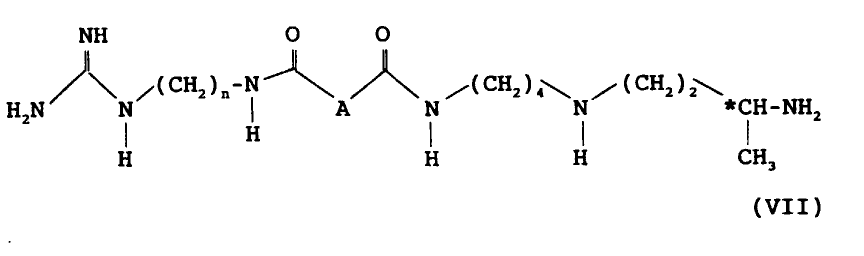 wherein A represents a single bond, -CH₂-, -CH₂O-, -CH₂NH-, -CH(OH)-, CHF- or -CH(OCH₃)-; n represents an integer of 6 to 8; and ∗C represents a carbon atom having (R) or (R,S) configuration; or salts thereof.

Furthermore, the spergualin-related compounds are described in Japanese Patent PCT KOKAI No. 9-511765 and shown by general formula (VIII): 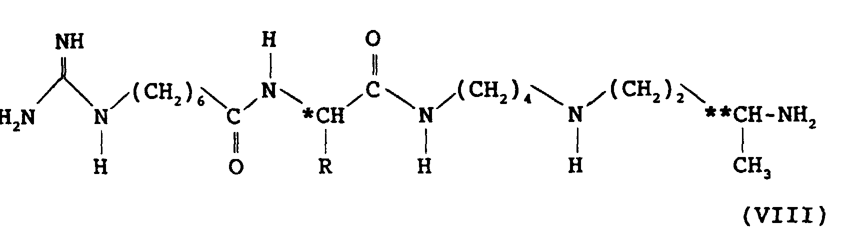 wherein R represents a hydrogen atom, OH group, OCH₃ group or CH₂OH group; ∗C represents an asymmetric carbon atom of (R,S), (R) or (S) configuration when R is not a hydrogen atom; and ∗∗C represents an asymmetric carbon atom of (R,S) or (R) configuration; and salts thereof.

The spergualin-related compounds described above can be synthesized by conventional methods disclosed in the respective specifications supra.

The salts of these spergualin-related compounds can be any salts of inorganic and organic acids as long as they are pharmacologically acceptable. Preferred examples of the inorganic acids include hydrochloric acid, sulfuric acid, nitric acid and phosphoric acid; and preferred examples of the organic acids are acetic acid, propionic acid, succinic acid, fumaric acid, maleic acid, malic acid, tartaric acid, glutaric acid, citric acid, benzenesulfonic acid, toluenesulfonic acid, methanesulfonic acid, ethanesulfonic acid, propanesulfonic acid, aspartic acid, glutamic acid, etc.

In the present invention, immunodeficiency diseases with reduction in a CD4⁺ T cell level are, e.g., immunodeficiency disease caused by HIV infections.

The immunosuppressive compound which is the effective ingredient in the present invention can be used for treating immunodeficiency diseases. In this case, the immunosuppressive compound is administered alone or by mixing with an excipient or with an carrier, parenterally, orally or percutaneously or, in the form of a suppository. The excipient and carrier are appropriately chosen from pharmaceutically acceptable compounds. The kind and composition vary depending upon route or method for administration. For example, water, alcohols, animal or vegetable oil such as soybean oil, peanut oil, sesame oil, mineral oil, etc., or synthetic oil can be used as a liquid carrier. Appropriate examples of solid carrier are sugars such as lactose, maltose, sucrose, etc., amino acids, cellulose derivatives such as hydroxypropylcellulose, etc., organic acid salts such as magnesium stearate.

For example, Japanese Patent KOKAI No. 61-165322 discloses injection formulation. This known formulation is applicable to that of the present invention. As an excipient for injection, there are known mannitol, maltose, dextran, lactose, cyclodextrin, chondroitin sulfate, gelatin, human serum albumin, etc. Particularly preferred are maltose, lactose, chondroitin sulfate, gelatin and human serum albumin. Where a lyophilized preparation is prepared, the effective ingredient of the present invention and these excipients are dissolved in sterile water, a pH of the resulting solution is adjusted to a range of 2 to 6, preferably 3 to 5 and the solution is then freeze dried. The preparation may be administered for use by dissolving in an appropriate solution for injection, e.g., a solution for intravenous administration such as an aqueous solution for injection, physiological saline, dextrose in water, an electrolyte solution, an amino acid solution, etc.

The composition of the present invention may be percutaneously administered. For this purpose, the method for administration and preparation disclosed in, e.g., Japanese Patent KOKAI No. 7-285857, WO 96/00058, etc. are available. For percutaneous administration, there is constructed a device comprising a donor electrode containing the effective ingredient of the present invention and suitable carriers, a counter electrode and a power source, wherein the donor electrode and the counter electrode are connected to the power source, respectively. The donor and counter electrodes of the device are applied to the skin of a patient.

A representative example of the preparation for percutaneous administration comprises a dissolving agent such as polyethylene glycol-glycerine complex, etc., a percutaneous absorption promoter and an emulsifying agent.

In the present invention, the preparations described above may further contain an acid, an alkali or an optimum amount of buffering agent for the purpose of adjusting pH, etc.

The amount of the effective ingredient in the preparations vary depending on the form of preparation but is generally in a range of 0.01 to 100 wt%, preferably 0.1 to 98 wt%. In the case of injection, the preparation may advantageously contain the effective ingredient generally in an amount of 0.1 to 30 wt%, preferably 1 to 10 wt%.

An appropriate dose is determined depending on age, body weight and conditions of a patient, purpose for the treatment, route for administration, etc., but in general, the therapeutic dose is 0.01 to 100 mg/kg·day for parenteral administration and 0.1 to 500 mg/kg·day for oral administration.

The immunosuppressive compounds of the present invention are characteristic of their relatively low toxicity and less accumulation of the toxicity even when the compounds are consecutively administered. Where the compounds of the present invention are intraperitoneally administered in a single dose to mice, the 50% lethal dose, i.e., LD₅₀ is 25 to 50 mg/kg.

The therapeutic effect of 15-deoxyspergualin for immunodeficiency was clinically investigated by administering the compound to HIV-infected patients. The details are shown below.

### Test Example 1

To a white male patient of 38 years old who was diagnosed as a HIV positive 1 year and 10 months ago, the 15-deoxyspergualin injection of Example 1 later described was intravenously administered for consecutive 15 days in a dose of 0.15 mg/kg body weight/day. This cycle was made one course. Then, no drug period was set for 6 weeks followed by repetition with 2 courses with the same interval between the second course and the third couse, as well. Prior to the treatment, the patient complained of fatigue (malaise) of the worst kind and showed a CD4⁺/CD8⁺ level of 0.68. However, no sign of other conditions related to AIDS was observed at all.

After Course 1 of the treatment was finished, the complaint of fatigue disappeared completely. The CD4⁺/CD8⁺ level was measured immediately before and after each course and, 3 months and 2 years after the completion of Course 3, namely, 8 measurements in total. The results are shown below.

### Results:

| | | | |
|---|---|---|---|
| Immediately before Course 1 | 0.68 | Immediately after Course 1 | 0.72 |
| Immediately before Course 2 | 0.78 | Immediately after Course 2 | 0.75 |
| Immediately before Course 3 | 0.87 | Immediately after Course 3 | 0.84 |
| 3 Months after completion of Course 3 | 0.93 | 2 Years after completion of Course 3 | 0.90 |

As is clearly noted from the results above, the CD4⁺/CD8⁺ level was gradually returned to the normal level (1.0) after the treatment was initiated and, the level reached was basically maintained for the following 2 years, with complete disappearance of the fatigue. It is thus evident that administration of 15-deoxyspergualin suppressed the development of AIDS.

In the results, the two levels immediately after Courses 2 and 3 decreased by 0.03 than those immediately before the courses. The decrease is considered to be due to a weak bone marrow suppression action (side effect) of 15-deoxyspergualin. The side effect can be eliminated by termination of administrating the drug. Thus, after the drug administration was terminated, the CD4⁺/CD8⁺ level was on the way of returning to the normal level. Throughout the entire period of the treatment, leucocyte count was maintained to approximately 6200 and no other side effect was observed at all. No drug other than 15-deoxyspergualin was administered at all throughout the treatment period.

### Test Example 2

To five (5) carriers diagnosed as HIV positive, the 15-deoxyspergualin injection was intravenously administered for consecutive 14 days in a dose of 0.15 mg/kg body weight/day. This cycle was made one course. Then, no drug period was set for 6 weeks followed by repetition with 2 courses with the same interval between the second course and the third course, as well. The CD4⁺ cell count, CD4⁺ /CD8⁺ level and virus load, etc. were measured immediately before and after each course and 6 weeks after the completion of Course 3, respectively. The results are shown below.

### Case 1:

A female patient of 23 years old diagnosed as HIV positive (1994, 3 years before) complained of fatigue (malaise) of the worst kind but no other AIDS-associated symptoms were observed.

| CD4⁺ T cell counts/µl | | | |
|---|---|---|---|
| Immediately before Course 1 | 680 | Immediately after Course 1 | 680 |
| Immediately before Course 2 | 730 | Immediately after Course 2 | 740 |
| Immediately before Course 3 | 720 | Immediately after Course 3 | 750 |
| 6 Weeks after completion of Course 3 | 760 | | |

| CD4⁺/CD8⁺ levels | | | |
|---|---|---|---|
| Immediately before Course 1 | 0.78 | Immediately after Course 1 | 0.82 |
| Immediately before Course 2 | 0.80 | Immediately after Course 2 | 0.86 |
| Immediately before Course 3 | 0.85 | Immediately after Course 3 | 0.93 |
| 6 Weeks after completion of Course 3 | 0.93 | | |

| Virus loads: particles/ml | | | |
|---|---|---|---|
| Immediately before Course 1 | 12000 | Immediately after Course 1 | 8600 |
| Immediately before Course 2 | 9000 | Immediately after Course 2 | 8000 |
| Immediately before Course 3 | 8200 | Immediately after Course 3 | 7000 |
| 6 Weeks after completion of Course 3 | 6000 | | |

### Case 2:

A female patient of 34 years old diagnosed as HIV positive (1992, 5 years before) had been complaining of fatigue (malaise) since 1992 but no other AIDS-like symptoms were noted.

| CD4⁺ T cell counts/µl | | | |
|---|---|---|---|
| Immediately before Course 1 | 750 | Immediately after Course 1 | 740 |
| Immediately before Course 2 | 750 | Immediately after Course 2 | 730 |
| Immediately before Course 3 | 760 | Immediately after Course 3 | 750 |
| 6 Weeks after completion of Course 3 | 740 | | |

| CD4⁺/CD8⁺ levels | | | |
|---|---|---|---|
| Immediately before Course 1 | 0.76 | Immediately after Course 1 | 0.72 |
| Immediately before Course 2 | 0.78 | Immediately after Course 2 | 0.77 |
| Immediately before Course 3 | 0.80 | Immediately after Course 3 | 0.79 |
| 6 Weeks after completion of Course 3 | 0.78 | | |

| Virus loads: particles/ml | | | |
|---|---|---|---|
| Immediately before Course 1 | 15000 | Immediately after Course 1 | 13000 |
| Immediately before Course 2 | 13000 | Immediately after Course 2 | 14000 |
| Immediately before Course 3 | 15000 | Immediately after Course 3 | 14000 |
| 6 Weeks after completion of Course 3 | 13000 | | |

### Case 3:

A male patient of 45 years old diagnosed as HIV positive (1996, 1 year before) complained of fatigue (malaise) of the worst kind but no other AIDS-associated symptoms were observed.

| CD4⁺ T cell counts/µl | | | |
|---|---|---|---|
| Immediately before Course 1 | 550 | Immediately after Course 1 | 570 |
| Immediately before Course 2 | 590 | Immediately after Course 2 | 600 |
| Immediately before Course 3 | 650 | Immediately after Course 3 | 720 |
| 6 Weeks after completion of Course 3 | 800 | | |

| CD4⁺/CD8⁺ levels | | | |
|---|---|---|---|
| Immediately before Course 1 | 0.69 | Immediately after Course 1 | 0.73 |
| Immediately before Course 2 | 0.72 | Immediately after Course 2 | 0.75 |
| Immediately before Course 3 | 0.84 | Immediately after Course 3 | 0.90 |
| 6 Weeks after completion of Course 3 | 0.90 | | |

| Virus loads: particles/ml | | | |
|---|---|---|---|
| Immediately before Course 1 | 17000 | Immediately after Course 1 | 11000 |
| Immediately before Course 2 | 11000 | Immediately after Course 2 | 7000 |
| Immediately before Course 3 | 8000 | Immediately after Course 3 | 6000 |
| 6 Weeks after completion of Course 3 | 6000 | | |

### Case 4:

A male patient of 40 years old diagnosed as HIV positive (1995, 2 years before) complained of fatigue (malaise) of the worst kind but no other AIDS-associated symptoms were observed.

| CD4⁺ cell counts/µl | | | |
|---|---|---|---|
| Immediately before Course 1 | 640 | Immediately after Course 1 | 710 |
| Immediately before Course 2 | 770 | Immediately after Course 2 | 760 |
| Immediately before Course 3 | 840 | Immediately after Course 3 | 850 |
| 6 Weeks after completion of Course 3 | 910 | | |

| CD4⁺/CD8⁺ levels | | | |
|---|---|---|---|
| Immediately before Course 1 | 0.73 | Immediately after Course 1 | 0.75 |
| Immediately before Course 2 | 0.83 | Immediately after Course 2 | 0.85 |
| Immediately before Course 3 | 0.90 | Immediately after Course 3 | 0.94 |
| 6 Weeks after completion of Course 3 | 0.92 | | |

| Virus loads: particles/ml | | | |
|---|---|---|---|
| Immediately before Course 1 | 25000 | Immediately after Course 1 | 20000 |
| Immediately before Course 2 | 20000 | Immediately after Course 2 | 18000 |
| Immediately before Course 3 | 17000 | Immediately after Course 3 | 15000 |
| 6 Weeks after completion of Course 3 | 13000 | | |

### Case 5:

A female patient of 37 years old diagnosed as HIV positive (1995, 2 years before) complained of fatigue (malaise) of the worst kind but no other AIDS-associated symptoms were observed.

| CD4⁺ cell counts/µl | | | |
|---|---|---|---|
| Immediately before Course 1 | 780 | Immediately after Course 1 | 750 |
| Immediately before Course 2 | 810 | Immediately after Course 2 | 820 |
| Immediately before Course 3 | 880 | Immediately after Course 3 | 890 |
| 6 Weeks after completion of Course 3 | 900 | | |

| CD4⁺/CD8⁺ levels | | | |
|---|---|---|---|
| Immediately before Course 1 | 0.79 | Immediately after Course 1 | 0.80 |
| Immediately before Course 2 | 0.81 | Immediately after Course 2 | 0.83 |
| Immediately before Course 3 | 0.85 | Immediately after Course 3 | 0.87 |
| 6 Weeks after completion of Course 3 | 0.90 | | |

| Virus loads: particles/ml | | | |
|---|---|---|---|
| Immediately before Course 1 | 6000 | Immediately after Course 1 | 6000 |
| Immediately before Course 2 | 7000 | Immediately after Course 2 | 6500 |
| Immediately before Course 3 | 6500 | Immediately after Course 3 | 6000 |
| 6 Weeks after completion of Course 3 | 6000 | | |

In terms of the conditions, the fatigue (malaise) of the worst kind was improved in Cases 1, 3 and 4 and no change was noted in Cases 2 and 5.

Hereinafter the present invention will be described below, with reference to the pharmaceutical preparations but is not deemed to be limited thereto.

### Example 1

### Injection:

By adding to a mixture of 10 parts by weight of 15-deoxyspergualin and 20 parts by weight of lactose, 1N-hydrochloric acid (q.p.) and distilled water for injection (q.p.), pH was adjusted to 5.0 to give 500 parts by weight of the solution. After the pH-adjusted solution was sterilized and filtered through a membrane filter, the filtrate was dispensed into glass containers for injection followed by freeze drying. Thus. freeze-dried injections containing 100 mg of 15-deoxyspergualin hydrochloride per vial were obtained.

### Industrial Applicability

The spergualin-related compounds such as 15-deoxyspergualin exhibit an excellent effect of improving CD4⁺/CD8⁺ level of patients with immunodeficiency accompanied by reduced CD4⁺ T cells. These compounds are less toxic and highly safe. Therefore, these compounds are useful for the treatment of immunodeficiency accompanied by a reduced CD4⁺ T cell level.

## Claims

1. A pharmaceutical composition for the treatment of immunodeficiency disease accompanied by a reduced CD4⁺ T cell level, comprising as an effective ingredient an immunosuppressive compound having an activity of suppressing the function of monocyte or macrophage, or a pharmacologically acceptable salt thereof.

2. A pharmaceutical composition according to claim 1, wherein said immunodeficiency disease accompanied by a reduced CD4⁺ T cell level is caused by human immunodeficiency virus (HIV) infection.

3. A pharmaceutical composition according to claim 1, wherein said immunosuppressive compound is spergualin or a spergualin-related compound having an immunosuppressive activity.

4. A pharmaceutical composition according to claim 3, wherein said spergualin-related compound having an immunosuppressive activity is 15-deoxyspergualin.

5. A pharmaceutical composition for treating HIV infection or for preventing the development of HIV infection, comprising as an effective ingredient 15-deoxyspergualin or a pharmacologically acceptable salt thereof.

6. Use of an immunosuppressive compound having the activity of suppressing the function of monocyte or macrophage for preparing a pharmaceutical composition for HIV infection accompanied by a reduced CD4⁺ T cell level, comprising as an effective ingredient said immunosuppressive compound or a pharmacologically acceptable salt thereof.

7. Use of a pharmaceutical composition for treating HIV infection or for preventing the development of HIV infection, comprising as an effective ingredient 15-deoxyspergualin or a pharmacologically acceptable salt thereof.

8. A method for treating an immunodeficiency disease accompanied by a reduced CD4⁺ T cell level, which comprises administering to the patient an effective dose of immunosuppressive compound having an activity of suppressing the function of monocyte or macrophage, or a pharmacologically acceptable salt thereof.

9. A method for treating HIV infection or preventing the development of HIV infection, which comprises administering to the patient an effective dose of 15-deoxyspergualin or a pharmacologically acceptable salt thereof.
